# EUROPÄISCHE PATENTANMELDUNG

(11) **EP 2 966 436 A1**
(43) Veröffentlichungstag der Anmeldung: **13.01.2016**
(21) Anmeldenummer: 15175393.6
(22) Anmeldetag: 06.07.2015
(51) Int. Cl.: G01N 22/00, G01N 33/36

(54) **Mikrowellenhohlraumresonator sowie damit ausgerüstete Spinnereivorbereitungsmaschine**

(30) Priorität: 10.07.2014 DE 102014109651
(71) Anmelder: Rieter Ingolstadt GmbH, 85055 Ingolstadt (DE)
(72) Erfinder: Hermann, Tobias, 80798 München (DE); Ueding, Michael, 85049 Ingolstadt (DE); Kovacs, Otmar, 92334 Berching (DE); Eibert, Prof. Dr. Thomas, 81925 München (DE); Faz, Muhammad Usman, 81245 München (DE); Siart, Dr. Uwe, 81735 München (DE)
(74) Vertreter: Baudler, Ron

(57) **Zusammenfassung**

Die Erfindung betrifft einen Mikrowellenhohlraumresonator (8) zur Überwachung eines strangförmigen Fasermaterials, wobei der Mikrowellenhohlraumresonator (8) eine innenliegende Resonatorkammer (7) aufweist, in dem mit Hilfe wenigstens einer Einkoppelanordnung (9) des Mikrowellenhohlraumresonators (8) ein elektrisches Feld erzeugbar ist, wobei der Mikrowellenhohlraumresonator (8) zumindest eine in die Resonatorkammer (7) mündende Eintrittsöffnung (10) für das Fasermaterial (3; 15) aufweist, über den das Fasermaterial (3; 15) in einer vorgegebenen Transportrichtung (T) in die Resonatorkammer (7) eintreten kann, wobei der Mikrowellenhohlraumresonator (8) zumindest eine Austrittsöffnung (11) für das Fasermaterial (3; 15) aufweist, und wobei der Mikrowellenhohlraumresonator (8) einen die Eintrittsöffnung (10) und die Austrittsöffnung (11) in der genannten Transportrichtung (T) verbindenden und durch die Resonatorkammer (7) verlaufenden Durchtrittskanal (13) für das Fasermaterial (3; 15) aufweist. Erfindungsgemäß wird vorgeschlagen, dass die Innenkontur der Resonatorkammer (7) derart ausgebildet ist, dass in einem parallel zur Transportrichtung (T) verlaufenden Querschnitt des Mikrowellenhohlraumresonators (8) die Orte mit gleichem Betrag der elektrischen Feldstärke jeweils auf Linien (14) liegen, deren Form im Bereich des Durchtrittskanals (13) eine in Richtung der Transportrichtung (T) verlaufende maximale räumliche Ausdehnung (A1) besitzt, die kleiner ist als ihre senkrecht hierzu verlaufende maximale räumliche Ausdehnung (A2).

## Beschreibung

Die vorliegende Erfindung betrifft einen Mikrowellenhohlraumresonator zur Überwachung der Dicke und/oder der Feuchtigkeit eines sich durch den Mikrowellenhohlraumresonator bewegenden strangförmigen Fasermaterials an einer Spinnereivorbereitungsmaschine, wobei der Mikrowellenhohlraumresonator eine innenliegende Resonatorkammer aufweist, in dem mit Hilfe wenigstens einer Einkoppelanordnung des Mikrowellenhohlraumresonators ein elektrisches Feld erzeugbar ist, wobei der Mikrowellenhohlraumresonator zumindest eine in die Resonatorkammer mündende Eintrittsöffnung für das Fasermaterial aufweist, über den das Fasermaterial in einer vorgegebenen Transportrichtung in die Resonatorkammer eintreten kann, wobei der Mikrowellenhohlraumresonator zumindest eine Austrittsöffnung für das Fasermaterial aufweist, über den das Fasermaterial nach Passieren der Resonatorkammer und in der genannten Transportrichtung wieder aus der Resonatorkammer austreten kann, und wobei der Mikrowellenhohlraumresonator einen die Eintrittsöffnung und die Austrittsöffnung in der genannten Transportrichtung verbindenden und durch die Resonatorkammer verlaufenden Durchtrittskanal für das Fasermaterial aufweist.

Darüber hinaus wird eine Spinnereivorbereitungsmaschine vorgeschlagen, wobei die Spinnereivorbereitungsmaschine vorzugsweise als Karde, Kämmmaschine oder Strecke ausgebildet ist.

Die Messung von Fasereigenschaften in der Textilindustrie ist eine unabdingbare Voraussetzung zur Produktion von hochwertigen Textilien. So ist beispielsweise die Messung der Dicke von strangförmigen Fasermaterialien, insbesondere zum Zwecke der Ausregulierung von Ungleichmäßigkeiten von einem oder mehreren der entsprechenden Spinnereivorbereitungsmaschine vorgelegten Faserbändern, unabdingbar. Gleichfalls ist zur Qualitätskontrolle des verstreckten Materials an einem Ausgang der Spinnereivorbereitungsmaschine eine derartige Messung wünschenswert. Messwerte zur Dicke des Fasermaterials (es sind auch die Bezeichnungen Bandquerschnitt oder Bandmasse gebräuchlich) werden neben der genannten Qualitätskontrolle auch zum Abstellen der Maschine herangezogen, wenn vorgegebene Grenzwerte überschritten werden und somit kein hochwertiges Produkt mehr produziert wird.

Bisher werden überwiegend mechanisch abtastende Sensoren zur Ermittlung der genannten Dicke des überwachten Fasermaterials eingesetzt. Auch sind kapazitive Messorgane bekannt.

Eine relativ neue Methode zur Messung bzw. Überwachung der Dicke eines Fasermaterials stellt die Verwendung von Mikrowellen dar. Hierbei werden von einem Mikrowellengenerator erzeugte Mikrowellen mit Hilfe einer Einkoppelanordnung in eine Resonatorkammer eines Mikrowellenhohlraumresonators eingekoppelt, durch den auch das zu vermessende Fasermaterial kontinuierlich hindurchgeführt wird. Entsprechend der Faserart, der Dicke des Fasermaterials und dessen Feuchte tritt bei einer charakteristischen Mikrowellenfrequenz ein Resonanzsignal auf, welches nach Auskopplung von einem Rechner zur Ermittlung der Fasermaterialdicke und/oder der Fasermaterialfeuchtigkeit auswertbar ist. Die Vorteile eines derartigen Messverfahrens mittels Mikrowellen liegen insbesondere darin, dass ein hochpräzises, berührungsloses Abtasten eines strangförmigen und schnelllaufenden Fasermaterials möglich ist. Mechanische Beeinträchtigungen des Fasermaterials sowie Messungenauigkeiten aufgrund der Trägheit von mechanischen Messelementen scheiden aus.

Das Messprinzip beruht grundsätzlich auf der Ausnutzung der dielektrischen Wechselwirkung zwischen dem durch den Durchtrittskanal durchlaufenden Fasermaterial und dem in der Resonatorkammer ausgebildeten hochfrequenten elektrischen Wechselfeld (im Folgenden als elektrisches Feld bezeichnet). Die Reaktion des Mikrowellenhohlraumresonators auf das Material (d. h. das Messsignal) geht dabei aus einer Integration über den gesamten mit dem elektrischen Feld durchsetzten Bereich des Durchtrittskanals hervor, dessen Ausdehnung in der vorgegebenen Transportrichtung im Wesentlichen der räumlichen Ausdehnung der Resonatorkammer in Transportrichtung des Fasermaterials entspricht.

Die bekannten Mikrowellenhohlraumresonatoren besitzen eine kreiszylindrische Resonatorkammer, wobei aufgrund der physikalischen Gesetzmäßigkeiten der Elektrodynamik ein umgekehrt proportionaler Zusammenhang zwischen der Resonanzfrequenz und dem Durchmesser der Resonatorkammer (und damit der Länge des Durchtrittskanals) besteht. Bei der bisher verwenden Zylinderbauform ist daher mit dem Durchmesser auch die Mindestlänge des Durchtrittskanals festgelegt.

Durch die Integration über diese große Distanz sind örtlich verteilte Masseschwankungen des durchlaufenden Fasermaterials nur mit relativ grober Ortsauflösung erfassbar. Das heißt, kleine Fehlerwellenlängen können gar nicht bzw. nur mit stark verringerter Empfindlichkeit erkannt und damit auch nicht sinnvoll im Rahmen des Maschinenprozesses eliminiert werden.

Das Problem besteht nun darin, dass grundsätzlich eine definierte Leerresonanzfrequenz des Resonators gefordert wird, die für die messtechnische Auswertung optimal ist. Hier haben sich typische Resonanzfrequenzen zwischen 2,5 GHz und 4 GHz als sinnvoll erwiesen. Daraus ergibt sich jedoch wiederum eine Mindestlänge des Durchtrittskanals.

Eine Erhöhung der Leerresonanzfrequenz führt zwar zu einer Verkleinerung des Durchmessers der Resonatorkammer, einer Verkürzung des Durchtrittskanals und damit unmittelbar zu einer besseren Ortsauflösung. Die Leerresonanz kann jedoch aus physikalischen bzw. technischen Gründen nicht beliebig erhöht werden.

Aufgabe der vorliegenden Erfindung ist es daher, einen Mikrowellenhohlraumresonator vorzuschlagen, der es ermöglicht, eine vorgegebene niedrige Resonanzfrequenz zu nutzen und trotzdem eine ausreichende Ortsauflösung der Messung sicherzustellen.

Die Aufgabe wird gelöst durch einen Mikrowellenhohlraumresonator mit den Merkmalen von Anspruch 1.

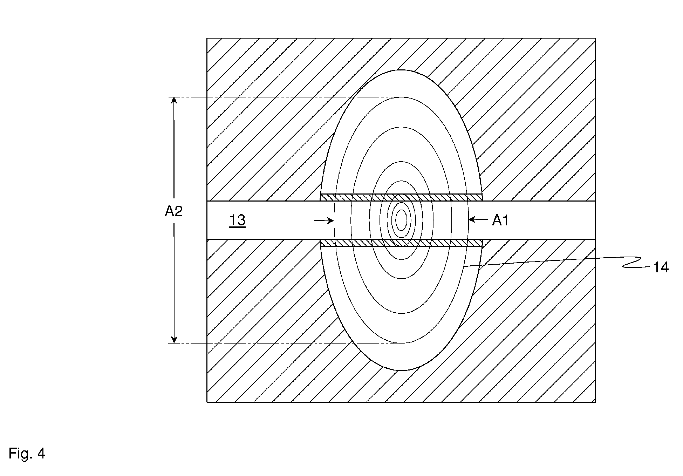

Erfindungsgemäß zeichnet sich der Mikrowellenhohlraumresonator nun dadurch aus, dass die Innenkontur der Resonatorkammer derart ausgebildet ist, dass in einem parallel zur Transportrichtung verlaufenden Querschnitt des Mikrowellenhohlraumresonators (vgl. beispielsweise Figur 4) die Orte mit gleichem Betrag der elektrischen Feldstärke jeweils auf Linien liegen, deren Form (d. h. deren räumliche Gestalt) im Bereich des Durchtrittskanals eine in Richtung der Transportrichtung verlaufende maximale räumliche Ausdehnung besitzt, die kleiner ist als ihre senkrecht hierzu verlaufende maximale räumliche Ausdehnung.

Im Übrigen kann es sich bei einzelnen oder allen der genannten Linien um geschlossene Linien, d. h. um Linien ohne Anfang und ohne Ende, handeln, so dass die Form der entsprechenden Linien jeweils den Umriss eines zweidimensionalen Körpers darstellt (mathematisch ausgedrückt stellen die Linien in diesem Fall also geschlossene ebene Kurven dar, die eine Form mit entsprechenden räumliche Ausdehnungen besitzt). Ebenso können einzelne oder alle Linien auch als nicht geschlossene Linien (mathematisch: "offene ebene Kurven") vorliegen, die jeweils einen Anfang und ein davon beabstandetes Ende besitzen. Auch in diesem Fall gilt jedoch, dass die entsprechenden Linien eine geometrische Form aufweisen, die den im vorangegangenen Absatz genannten Vorgaben entspricht.

Mit anderen Worten ist die Resonatorkammer also derart gestaltet, dass es im Bereich des Durchtrittskanals zu einer räumlichen Konzentration der genannten Linien in Transportrichtung kommt, so dass der Abstand benachbarter Linien in Transportrichtung kleiner ist als der entsprechende Abstand in einer senkrecht hierzu verlaufenden Richtung. Die genannten Linien besitzen beispielsweise eine Form eines in Transportrichtung gestauchten Kreises bzw. einer entsprechend gestauchten Ellipse. In jedem Fall ist durch die entsprechende Innenkontur der Resonatorkammer, die sich unmittelbar auf die Form der genannten Linien auswirkt, sichergestellt, dass es im Bereich des Durchtrittskanals (der im Übrigen in einem senkrecht zur Transportrichtung verlaufenden Schnitt zylindrisch, quadratisch oder rechteckig ausgebildet sein kann) eine gegenüber den restlichen Bereichen der Resonatorkammer zu einer Erhöhung der Intensität des elektrischen Feldes kommt. Der Durchtrittskanal kann hierdurch gegenüber einer zylindrischen Resonatorkammer in der genannten Transportrichtung bei ansonsten gleichbleibender Resonatorfrequenz (bevorzugt werden Frequenzen zwischen 2 GHz und 5 GHz) signifikant verkürzt werden, so dass die Ortsauflösung entsprechend verbessert ist.

An dieser Stelle sei allgemein darauf hingewiesen, dass die Resonatorkammer derart ausgebildet sein sollte, dass die Feldstärke in einer senkrecht zu den Ebenen, die durch die genannten Linien definiert werden, verlaufenden Richtung keinen Schwankungen unterworfen ist, um auch die Dicke und/oder Feuchte von mehreren nebeneinander durch den Durchtrittskanal laufenden Faserbändern gleichzeitig überwachen zu können.

Insbesondere ist es äußert vorteilhaft, wenn die Resonatorkammer in einem parallel zu den genannten Linien verlaufenden Querschnitt zwei beidseitig an den Durchtrittskanal angrenzende Bereiche aufweist. Die Bereiche können miteinander in Verbindung stehen. Denkbar ist jedoch auch, dass es sich bei den Bereichen um räumlich abgeschlossene und voneinander durch den Durchtrittskanal separierte Hohlräume handelt, wobei sich das elektrische Feld in beide Bereiche der Resonatorkammer erstreckt (der Durchtrittskanal kann hierfür beispielsweise, wie später noch ausführlicher beschrieben, durch elektrisch nicht leitende Kanalwandungsabschnitte begrenzt sein, die eine räumliche Trennung der genannten Bereiche bewirken).

In jedem Fall ist es von Vorteil, wenn die Bereiche jeweils abschnittsweise durch elektrisch leitende Wandabschnitte begrenzt sind, deren Innenkontur in dem genannten Querschnitt (d. h. parallel zu den durch die genannten Linien definierten Ebenen) von der Form eines Kreisbogens abweicht. Dies bewirkt schließlich die erfindungsgemäße Form der genannten Linien, die maßgeblich von der Form bzw. Anordnung der elektrisch leitenden Wandabschnitte abhängt. Im Übrigen sei an dieser Stelle darauf hingewiesen, dass die Form der genannten Innenkontur in sämtlichen, parallel zu den durch die genannten Linien gebildeten Ebenen verlaufenden, Querschnitten gleich sein sollte.

Besondere Vorteile bringt es mit sich, wenn die Innenkontur der elektrisch leitenden Wandabschnitte in dem genannten Querschnitt zumindest abschnittsweise geradlinig verläuft. Die elektrisch leitenden Wandabschnitte können beispielsweise eben ausgebildet sein und Innenflächen der Resonatorkammer bilden, die sich senkrecht zur Transportrichtung erstrecken. Einzelne der Wandabschnitte können hierbei parallel zueinander verlaufen oder einen rechten bzw. spitzen Winkel miteinander einschließen. Beispielsweise könnte der Mikrowellenhohlraumresonator zwei Wandabschnitte aufweisen, die die Resonatorkammer beidseitig begrenzen und parallel zu den genannten Linien verlaufen. Ebenso könnten Wandabschnitte vorhanden sein, die die Resonatorkammer beidseitig in einer senkrecht zu den genannten Linien verlaufenden Richtung begrenzen, wobei auch diese Wandabschnitte parallel zueinander und zudem, wenigstens abschnittsweise, parallel zur Transportrichtung verlaufen könnten.

Des Weiteren ist es vorteilhaft, wenn die Innenkontur der elektrisch leitenden Wandabschnitte der beiden Bereiche in dem genannten Querschnitt jeweils eine C-förmige Form aufweist. Die C-Form besteht prinzipiell aus einem ersten und einem zweiten Abschnitt und einem die genannten Abschnitte verbindenden dritten Abschnitt. Die jeweiligen Abschnitte können hierbei gebogen sein, so dass die Form einem "C" der lateinischen Schrift entspricht. Ebenso ist es denkbar, dass die genannten Abschnitte geradlinig bzw. abgeknickt verlaufen, so dass die Innenkontur beispielsweise einem Rechteck mit einer Unterbrechung im Bereich einer seiner beiden Längsseiten entsprechen kann. Insbesondere ist es von Vorteil, wenn die Innenkontur beider Bereiche prinzipiell einem Rechteck entspricht, wobei die beiden benachbart zueinander verlaufenden und den Durchtrittskanal teilweise begrenzenden Längsseiten jeweils eine Unterbrechung aufweisen. Die zuletzt genannten Längsseiten könnten beispielsweise durch Wandabschnitte gebildet sein, die plattenförmig ausgebildet sind und jeweils von außen in die Resonatorkammer hineinragen und diese damit in die zwei Bereiche unterteilen, wobei die Unterbrechungen durch elektrisch nicht leitende Kanalwandungsabschnitte überbrückt sein sollten, um die Bereiche nach außen abzuschließen und damit ein Eindringen von Schmutz zu verhindern.

Vorteilhaft ist es zudem, wenn die Innenkontur der elektrisch leitenden Wandabschnitte der beiden Bereiche in dem genannten Querschnitt bezüglich des Durchtrittskanals spiegelsymmetrisch ausgebildet ist. Hierdurch entsteht ein bezüglich des Durchtrittskanals spiegelsymmetrisches elektrisches Feld, das eine besonders genaue Überwachung des durchlaufenden Fasermaterials verspricht. Ebenso kann es von Vorteil sein, wenn die Innenkontur der elektrisch leitenden Wandabschnitte in einem senkrecht zu den genannten Linien verlaufenden Schnitt spiegelsymmetrisch verlaufen.

Auch ist es von Vorteil, wenn die elektrisch leitenden Wandabschnitte der beiden Bereiche der Resonatorkammer in dem genannten Querschnitt jeweils eine (bereits oben genannte) Unterbrechung aufweisen, wobei die Unterbrechungen jeweils von einem elektrisch nicht leitenden und den Durchtrittskanal zumindest abschnittsweise begrenzenden Kanalwandungsabschnitt überbrückt sind. Die genannten Kanalwandungsabschnitte können in einem senkrecht zur Transportrichtung verlaufenden Schnitt kreis- oder ellipsenförmig sein, so dass der Durchtrittskanal prinzipiell die Form eines Zylinders mit kreis- bzw. ellipsenförmigen Querschnitt aufweisen kann. Ebenso ist es denkbar, dass die elektrisch nicht leitenden Kanalwandungsabschnitte ebene Flächen ausbilden, der Durchtrittskanal in einem senkrecht zur Transportrichtung verlaufenden Schnitt also die Form eines Quadrats oder Rechtecks besitzt. Der Durchtrittskanal kann also eine Quaderform aufweisen, die sich in Transportrichtung durch den Mikrowellenhohlraumresonator erstreckt, so dass auch mehrere strangförmige Fasermaterialien nebeneinander durch den Mikrowellenhohlraumresonator geführt und dabei bezüglich Dicke und/oder Feuchtigkeit überwacht werden können.

Ebenso ist es vorteilhaft, wenn die elektrisch nicht leitenden Kanalwandungsabschnitte in dem genannten Querschnitt eine parallel zur Transportrichtung verlaufende Breite aufweisen, die kleiner ist als die senkrecht zur Transportrichtung und parallel zu den genannten Linien verlaufende maximale Höhe der Resonatorkammer. Da die Höhe durch die vorgegebene Resonanzfrequenz vorbestimmt ist, kann durch eine geringe Breite der nicht leitenden Kanalwandungsabschnitte (durch die sich das elektrische Feld in bzw. durch den Durchtrittskanal erstreckt und damit mit dem durchlaufenden Fasermaterial wechselwirken kann) eine erhöhte Ortsauflösung im Bereich des Durchtrittskanals erreicht werden, da es durch die geringe Breite zu einer Konzentration der Intensität des elektrischen Felds im Bereich des Durchtrittskanals kommt.

Des Weiteren ist es vorteilhaft, wenn die maximale Höhe der Resonatorkammer einen Betrag aufweist, der zwischen 80 mm und 200 mm, bevorzugt zwischen 100 mm und 180 mm, liegt. Hierdurch kann der Resonator mit einer Resonatorfrequenz zwischen 2 GHz und 5 GHz betrieben wird, da diese unmittelbar mit der Höhe der Resonatorkammer zusammenhängt. Die Resonatorkammer kann die genannte Höhe im Übrigen an mehreren Stellen aufweisen, insbesondere, wenn die die Höhe definierenden Wandungsabschnitte flächig ausgebildet und parallel zueinander und parallel zur Transportrichtung verlaufen.

Vorteilhaft ist es zudem, wenn die oben genannte Breite der elektrisch nicht leitenden Kanalwandungsabschnitte einen Betrag aufweist, der zwischen 20 mm und 80 mm, bevorzugt zwischen 30 mm und 70 mm, liegt. Da die Breite auch den Abstand der genannten Linien im Bereich des Durchtrittskanals bestimmt, hat sich die genannte Breite bewährt, um die gewünschte hohe Ortsauflösung im Bereich des Durchtrittskanals zu ermöglichen. Durch die geringe Breite bei Einhaltung der oben genannten Höhe der Resonatorkammer entsteht schließlich ein elektrisches Feld, dessen Intensität im Bereich des Durchtrittskanals gegenüber den restlichen Bereichen der Resonatorkammer erhöht ist. Im Ergebnis können damit auch kurzzeitige Schwankungen der Dicke und/oder Feuchtigkeit des durchlaufenden Fasermaterials zuverlässig erkannt werden, ohne dass die Resonatorfrequenz von dem oben genannten Bereich abweichen müsste, wie dies bei im Stand der Technik bekannten kreisförmigen Linien der Fall wäre, auf denen jeweils die Orte liegen, deren elektrische Feldstärke den gleichen Betrag aufweisen.

Besonders vorteilhaft ist es, wenn die Innenkonturen der beiden Bereiche der Resonatorkammer in dem genannten Querschnitt jeweils die Form eines Polygons aufweisen, wobei ein Teil des Polygons durch die Innenkontur einzelner elektrisch leitender Wandabschnitte und der restliche Teil des Polygons durch einen elektrisch nicht leitenden Kanalwandungsabschnitt gebildet ist. Vorzgusweise umfasst die genannte Innenkontur zwei zum Durchtrittskanal bzw. dessen Mittelachse spiegelsymmetrisch verlaufende Polygone, die jeweils die Form eines Rechtecks oder eines Trapezes aufweisen.

Vorteilhaft ist es, wenn die Innenkonturen der beiden Bereiche der Resonatorkammer jeweils quader- oder trogförmig ausgebildet sind, wobei die jeweiligen Bereiche durch die oben beschriebenen elektrisch leitenden Wandabschnitte und die jeweiligen elektrisch nicht leitenden Kanalwandungsabschnitte begrenzt sein sollten.

Die erfindungsgemäße Spinnereivorbereitungsmaschine zeichnet sich schließlich dadurch aus, dass sie zumindest einen Mikrowellenhohlraumresonator gemäß bisheriger bzw. nachfolgender Beschreibung aufweist. Bei der Spinnereivorbereitungsmaschine handelt es sich beispielsweise um eine aus dem Stand der Technik bekannte Kämmmaschine oder Karde. Besonders bevorzugt ist die Spinnereivorbereitungsmaschine jedoch als Strecke ausgebildet, wie sie beispielhaft in der nachfolgenden Figurenbeschreibung näher beschrieben ist. Der Mikrowellenhohlraumresonator kann sich in diesem Fall in einer für das zu verstreckende Fasermaterial vorgesehenen Förderrichtung der Strecke vor einem Streckwerk der Strecke befinden. Der Mikrowellenhohlraumresonator dient in diesem Fall der Überwachung der Dicke und/oder Feuchte des in das Streckwerk einlaufenden Fasermaterials. Auf Basis der vom Mikrowellenhohlraumresonator gelieferten Messwerte kann schließlich der Verzug des Streckwerks angepasst werden, um ein oder mehrere möglichst gleichmäßig verzogene Faserbänder zu erhalten. Ebenso ist es denkbar, den erfindungsgemäßen Mikrowellenhohlraumresonator im Bereich eines Ausgangs des Streckwerks zu platzieren, um das das Streckwerk verlassende Fasermaterial hinsichtlich Dicke und/oder Feuchtigkeit zu überwachen und damit sicherzustellen, dass das das Streckwerk verlassende Fasermaterial hinsichtlich der genannten Größen den vorgegebenen Sollwerten entspricht. In beiden Fällen kann der Mikrowellenhohlraumresonator mit einer Steuerung der Strecke verbunden sein, die während des Betriebs der Strecke auch eine Änderung des Verzugs des Streckwerks (basierend auf den Messdaten des bzw. der Mikrowellenhohlraumresonatoren) vornimmt.

Weitere Vorteile der Erfindung sind in den nachfolgenden Ausführungsbeispielen beschrieben. Es zeigen, jeweils schematisch:
- **Figur 1**: eine Seitenansicht einer als Strecke ausgebildeten Spinnereivorbereitungsmaschine,
- **Figur 2**: eine Perspektive eines Mikrowellenhohlraumresonators,
- **Figur 3**: einen parallel zur vorgegebenen Transportrichtung des durchlaufenden Fasermaterials verlaufenden Querschnitt eines erfindungsgemäßen Mikrowellenhohlraumresonators,
- **Figur 4**: die Ansicht gemäß Figur 3 ohne durchlaufendes Fasermaterial,
- **Figur 5**: einen parallel zur vorgegebenen Transportrichtung des durchlaufenden Fasermaterials verlaufenden Querschnitt eines weiteren erfindungsgemäßen Mikrowellenhohlraumresonators,
- **Figur 6**: die Ansicht gemäß Figur 5 ohne die Linien auf denen jeweils die Orte liegen, deren elektrische Feldstärke den gleichen Betrag aufweist, und
- **Figur 7**: einen parallel zur vorgegebenen Transportrichtung des durchlaufenden Fasermaterials verlaufenden Querschnitt eines weiteren erfindungsgemäßen Mikrowellenhohlraumresonators.

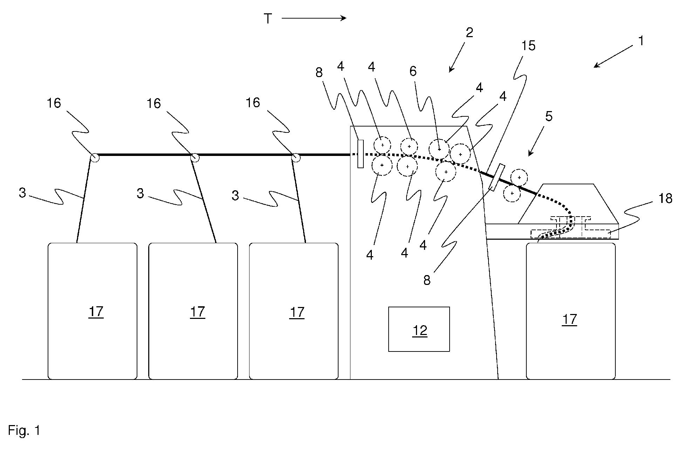

Figur 1 zeigt in einer Seitenansicht und als Beispiel für eine erfindungsgemäße Spinnereivorbereitungsmaschine 1 eine Strecke zum Verstrecken (Vergleichmäßigen) eines strangförmigen Fasermaterials in Form eines Faserverbands 3. Während des Betriebs der Strecke wird der Faserverband 3 (z. B. in Form von einzelnen Faserbändern) mit Hilfe einer Abzugsanordnung aus einer oder mehreren sogenannten Spinnkannen 17 entnommen und über entsprechende Umlenkungen 16 dem Streckwerk 2 der Strecke (bzw. im Fall einer Mehrkopfstrecke: den Streckwerken 2 der Strecke) zugeführt.

Das Streckwerk 2 besitzt eine Vielzahl von um entsprechende Drehachsen 6 (nur eine ist mit einem Bezugszeichen versehen) drehbare Streckwerkswalzen 4, durch die der Faserverband 3 klemmend geführt wird. Der gewünschte Verzug des Faserverbands 3 entsteht schließlich dadurch, dass die einzelnen zylinderförmigen Unterwalzen und damit auch die einzelnen, mit diesen in Kontakt stehenden, Oberwalzen, in der gezeigten Transportrichtung T des Faserverbands 3 eine zunehmend höhere Umfangsgeschwindigkeit besitzen. Durch in Transportrichtung T zunehmende Umfangsgeschwindigkeiten der jeweiligen Streckwerkswalzen 4 erfolgt schließlich ein Verstrecken und damit ein Vergleichmäßigen des Faserverbands 3.

Im Anschluss an das Streckwerk 2 wird das verstreckte Fasermaterial, das nun als Faservlies 15 vorliegt, durch einen nicht gezeigten Verdichter geleitet, der vorzugsweise als Vliestrichter ausgebildet ist und ein Verdichten des Faservlieses 15 bewirkt.

Anschließend gelangt das Faservlies 15 in den Bereich einer Abzugseinrichtung 5, die in der Regel mehrere drehbare bzw. zumindest teilweise angetriebene Abzugselemente, beispielsweise in Form zweier das Faservlies 15 von zwei Seiten kontaktierenden Abzugsscheiben, umfasst. Die Abzugseinrichtung 5 bewirkt durch eine entsprechend hohe Fördergeschwindigkeit einen weiteren Verzug und damit eine Erhöhung der Zugfestigkeit des Faservlieses 15. Schließlich wird das Faservlies 15 in der Regel einem rotierenden Drehteller 18 zugeführt und durch diesen schlingenförmig in eine bereitgestellte Spinnkanne 17 abgelegt, wobei die Steuerung der einzelnen Abschnitte mit Hilfe einer oder mehrerer Steuereinheiten 12 erfolgt.

Um nun den Verzug des Streckwerks 2 auf den zugeführten Faserverband 3 anpassen zu können, ist es insbesondere notwendig, Dickenschwankungen des Faserverbands 3 bereits vor dem Einlauf desselben in das Streckwerk 2 zu ermitteln. Hierzu besitzt die Strecke einen Mikrowellenhohlraumresonator 8, der im Bereich vor dem Streckwerk 2 angeordnet ist (sogenannter Einlaufresonator). Ebenso kann ein entsprechender Mikrowellenhohlraumresonator 8 in einem dem Streckwerk 2 in der genannten Transportrichtung T nachgeordneten Bereich platziert sein (sogenannter Auslaufresonator), um Dickenschwankungen des aus dem Streckwerk 2 auslaufenden Faservlieses 15 zu überwachen und damit Rückschlüsse auf einen nicht den Vorgaben entsprechenden Verzug des Streckwerks 2 zu ermöglichen (wobei der Verzug schließlich auf Basis der Messwerte des oder der Mikrowellenhohlraumresonatoren 8 mit Hilfe der Steuereinheit 12 angepasst werden kann).

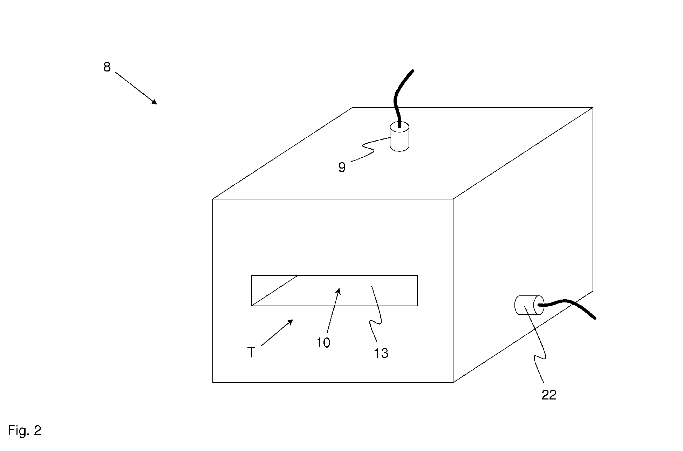

Eine perspektivische Ansicht eines entsprechenden Mikrowellenhohlraumresonators 8 zeigt Figur 2. Der Mikrowellenhohlraumresonator 8 besitzt prinzipiell eine Eintrittsöffnung 10, über die das dem Mikrowellenhohlraumresonator 8 zugeführte strangförmige Fasermaterial in eine innenliegende und in den folgenden Figuren noch näher dargestellte Resonatorkammer 7 gelangt. Ferner ist eine Austrittsöffnung 11 für das Fasermaterial vorgesehen, die sich im Bereich der hinteren, parallel zur Blattebene verlaufenden, Rückseite des Mikrowellenhohlraumresonators 8 befindet und daher in Figur 2 nicht sichtbar ist. Zwischen der Eintrittsöffnung 10 und der Austrittsöffnung 11 verläuft schließlich ein parallel zur Transportrichtung T verlaufender Durchtrittskanal 13 für das Fasermaterial, wobei dieser, wie in Figur 2 gezeigt, eine quaderförmige Form aufweisen kann.

Darüber hinaus besitzt der Mikrowellenhohlraumresonator 8 eine aus dem Stand der Technik bekannte und daher nicht näher beschriebene Einkoppelanordnung 9 zum Einkoppeln von Mikrowellen sowie eine entsprechende Auskoppelanordnung 22 für Mikrowellen. Ein Teil der Mikrowellen wird beim Betrieb des Mikrowellenhohlraumresonators 8 mit Hilfe der Auskoppelanordnung 22 aus der Resonatorkammer 7 ausgekoppelt, um daraus eine Resonanzkurve zu ermitteln. Dabei kann aus der Resonanzfrequenz und der Halbwertsbreite der Resonanzkurve auf die Masse und damit die Dicke und/oder die Feuchte des Fasermaterials geschlossen werden, da sich die beiden Parameter Resonanzfrequenz und Halbwertsbreite aufgrund der Wechselwirkung des Fasermaterials und des elektrischen Felds der Mikrowellen spezifisch einstellen.

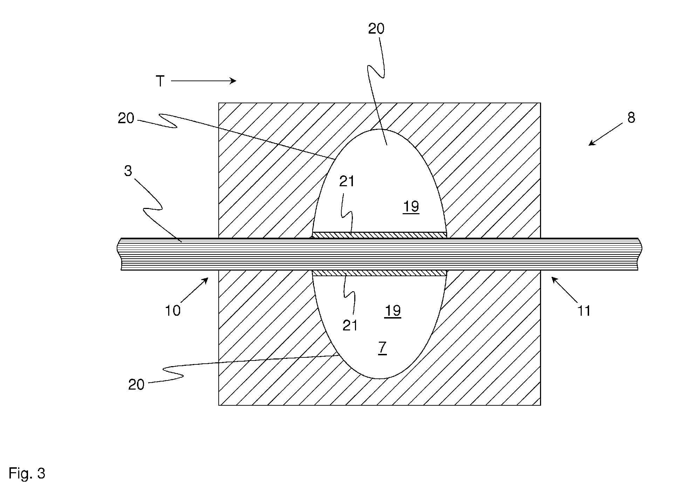

Figur 3 zeigt nun einen parallel zur Transportrichtung T verlaufenden Querschnitt eines erfindungsgemäßen Mikrowellenhohlraumresonators 8. Wie aus dieser Figur ersichtlich, setzt sich die Resonatorkammer 7 aus zwei durch den Durchtrittskanal 13 voneinander getrennte Bereiche 19 zusammen, wobei die beiden Bereich 19 jeweils durch mehrere elektrisch leitende Wandabschnitte 20 und einen den Durchtrittskanal 13 begrenzenden elektrisch nicht leitenden Kanalwandungsabschnitt 21 begrenzt werden (selbstverständlich besitzt der Mikrowellenhohlraumresonator 8 auch jeweils einen, bezogen auf die Blattebene, vorderen und einen hinteren elektrisch leitenden Wandabschnitt 20, durch die die Resonatorkammer 7 nach vorne und hinten verschlossen ist).

Wie nun Figur 4 zu entnehmen ist, ist die Innenkontur der Resonatorkammer 7 erfindungsgemäß derart gestaltet, dass das im Inneren der Resonatorkammer 7 erzeugte elektrische Feld parallel zur Blattebene verlaufende Linien 14 besitzt, wobei die Linien jeweils durch die Orte gebildet werden, deren elektrische Feldstärke den gleichen Betrag aufweisen, wobei die Form der Linien 14 (in Figur 4 prinzipiell elliptisch) im Bereich des Durchtrittskanals 13 eine in Richtung der Transportrichtung T verlaufende räumliche Ausdehnung A1 besitzt, die kleiner ist als ihre senkrecht hierzu verlaufende räumliche Ausdehnung A2 (wobei hinsichtlich der Bezugszeichen auch auf Figur 3 verwiesen wird, da in Figur 4 aus Übersichtsgründen nicht alle in beiden Figuren gleichsam gezeigten Abschnitte mit Bezugszeichen versehen sind). Dies führt im Bereich des Durchtrittskanals 13 zu einem relativ kurzen Wechselwirkungsbereich zwischen dem elektrischen Feld und dem durchlaufenden Fasermaterial (die lediglich im Bereich der elektrisch nicht leitenden Kanalwandungsabschnitte 21 erfolgen kann), so dass die Auflösung des Mikrowellenhohlraumresonators 8 gegenüber einer zylindrischen Resonatorkammer 7 deutlich erhöht ist.

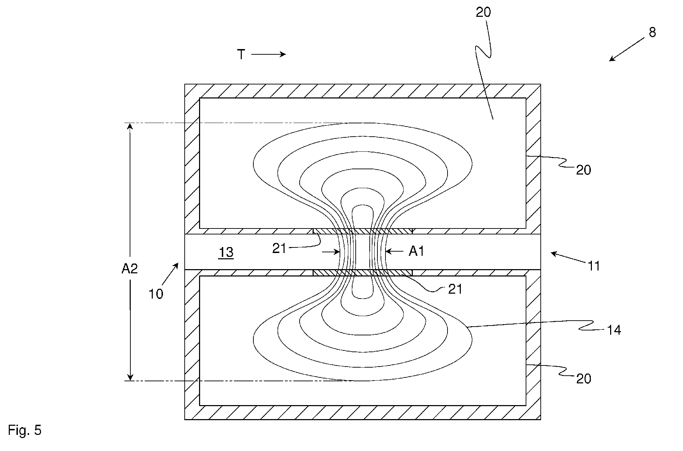

Eine weitere vorteilhafte Ausbildung der Innenkontur der Resonatorkammer 7 zeigt Figur 5. Die hier gezeigten elektrisch leitenden Wandabschnitte 20 sowie die diese verbindenden elektrisch nicht leitenden Kanalwandungsabschnitte 21 sind in diesem Fall jeweils eben ausgebildet, so dass die einzelnen Bereiche 19 der Resonatorkammer 7 eine quaderförmige Form aufweisen, wobei mit Ausnahme der parallel zur Blattebene verlaufenden Wandabschnitte 20 alle elektrisch leitenden Wandabschnitte 20 und auch die elektrisch nicht leitenden Kanalwandungsabschnitte 21 (durch die sich das elektrische Feld erstreckt) senkrecht zur Transportrichtung T verlaufen.

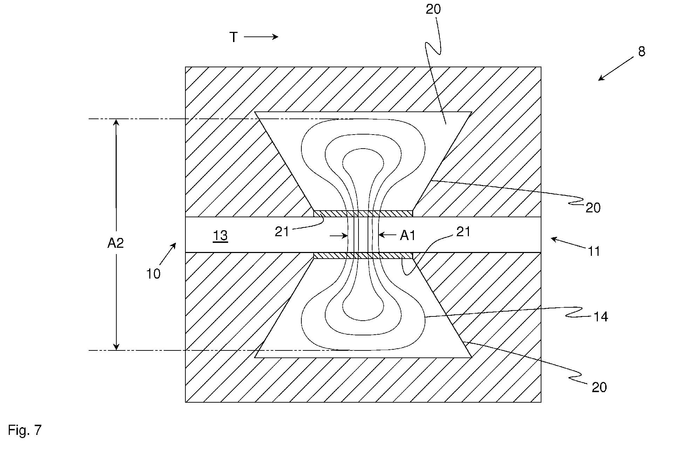

Schließlich zeigt Figur 7 eine letzte mögliche Ausführungsform der Innenkontur der Resonatorkammer 7. Die senkrecht zur Transportrichtung T verlaufenden elektrisch leitenden Wandabschnitte 20 bilden in diesem Fall gemeinsam mit den elektrisch nicht leitenden Kanalwandungsabschnitten 21 zwei spiegelsymmetrisch zum Durchtrittskanal 13 angeordnete Trapeze aus, die ebenfalls eine Form der genannten Linien 14 zur Folge haben, die im Bereich des Durchtrittskanals 13 eine in Richtung der Transportrichtung T verlaufende räumliche Ausdehnung A1 besitzt, die kleiner ist als ihre senkrecht hierzu verlaufende räumliche Ausdehnung A2.

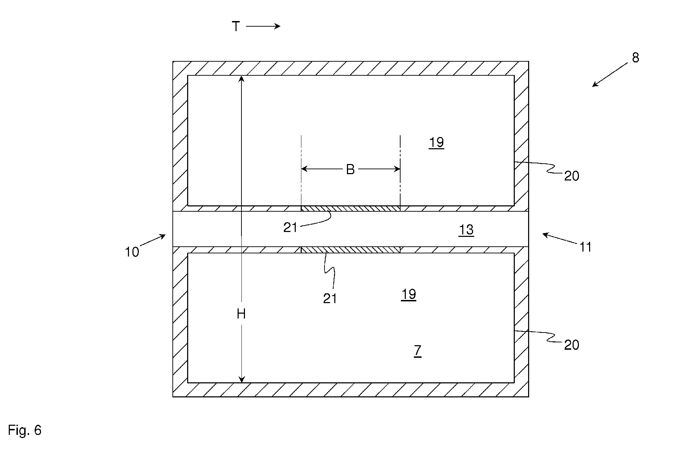

Im Übrigen sei an dieser Stelle darauf hingewiesen, dass die parallel zur Transportrichtung T verlaufende Breite B der elektrisch nicht leitende Kanalwandungsabschnitt kleiner sein sollte als die senkrecht hierzu verlaufende maximale Höhe H der Resonatorkammer 7 (die genannten Größen Breite B und Höhe H sind aus Übersichtsgründen nur in Figur 6 kenntlich gemacht).

Die vorliegende Erfindung ist nicht auf die dargestellten und beschriebenen Ausführungsbeispiele beschränkt. Abwandlungen im Rahmen der Patentansprüche sind ebenso möglich wie eine beliebige Kombination der beschriebenen Merkmale, auch wenn sie in unterschiedlichen Teilen der Beschreibung bzw. den Ansprüchen oder in unterschiedlichen Ausführungsbeispielen dargestellt und beschrieben sind, soweit sich die einzelnen Merkmale nicht widersprechen.

### Bezugszeichenliste

- 1: Spinnereivorbereitungsmaschine
- 2: Streckwerk
- 3: Faserverband
- 4: Streckwerkswalze
- 5: Abzugseinrichtung
- 6: Drehachse
- 7: Resonatorkammer
- 8: Mikrowellenhohlraumresonator
- 9: Einkoppelanordnung
- 10: Eintrittsöffnung des Resonatorraums
- 11: Austrittsöffnung des Resonatorraums
- 12: Steuereinheit
- 13: Durchtrittskanal für das strangförmige Fasermaterial
- 14: Linien konstanter Feldstärke
- 15: Faservlies
- 16: Umlenkung
- 17: Spinnkanne
- 18: Drehteller
- 19: Bereich des Resonatorraums
- 20: elektrisch leitender Wandabschnitt
- 21: elektrisch nicht leitender Kanalwandungsabschnitt
- 22: Auskoppelanordnung

- A1: in Richtung der Transportrichtung verlaufende räumliche Ausdehnung der durch eine Linie konstanter Feldstärke gebildeten Form
- A2: senkrecht zur Transportrichtung verlaufende räumliche Ausdehnung der durch eine Linie konstanter Feldstärke gebildeten Form
- B: parallel zur Transportrichtung verlaufende Breite des elektrisch nicht leitenden Kanalwandungsabschnitts
- H: maximale Höhe des Resonatorraums
- T: Transportrichtung

## Patentansprüche

1. Mikrowellenhohlraumresonator (8) zur Überwachung der Dicke und/oder der Feuchtigkeit eines sich durch den Mikrowellenhohlraumresonator (8) bewegenden strangförmigen Fasermaterials (3; 15) an einer Spinnereivorbereitungsmaschine (1),
- wobei der Mikrowellenhohlraumresonator (8) eine innenliegende Resonatorkammer (7) aufweist, in dem mit Hilfe wenigstens einer Einkoppelanordnung (9) des Mikrowellenhohlraumresonators (8) ein elektrisches Feld erzeugbar ist,
- wobei der Mikrowellenhohlraumresonator (8) zumindest eine in die Resonatorkammer (7) mündende Eintrittsöffnung (10) für das Fasermaterial (3; 15) aufweist, über den das Fasermaterial (3; 15) in einer vorgegebenen Transportrichtung (T) in die Resonatorkammer (7) eintreten kann,
- wobei der Mikrowellenhohlraumresonator (8) zumindest eine Austrittsöffnung (11) für das Fasermaterial (3; 15) aufweist, über den das Fasermaterial (3; 15) nach Passieren der Resonatorkammer (7) und in der genannten Transportrichtung (T) wieder aus der Resonatorkammer (7) austreten kann, und
- wobei der Mikrowellenhohlraumresonator (8) einen die Eintrittsöffnung (10) und die Austrittsöffnung (11) in der genannten Transportrichtung (T) verbindenden und durch die Resonatorkammer (7) verlaufenden Durchtrittskanal (13) für das Fasermaterial (3; 15) aufweist,
**dadurch gekennzeichnet,**
- **dass** die Innenkontur der Resonatorkammer (7) derart ausgebildet ist, dass in einem parallel zur Transportrichtung (T) verlaufenden Querschnitt des Mikrowellenhohlraumresonators (8) die Orte mit gleichem Betrag der elektrischen Feldstärke jeweils auf Linien (14) liegen, deren Form im Bereich des Durchtrittskanals (13) eine in Richtung der Transportrichtung (T) verlaufende maximale räumliche Ausdehnung (A1) besitzt, die kleiner ist als ihre senkrecht hierzu verlaufende maximale räumliche Ausdehnung (A2).

2. Mikrowellenhohlraumresonator (8) gemäß dem vorangegangenen Anspruch, **dadurch gekennzeichnet, dass** die Resonatorkammer (7) in einem parallel zu den genannten Linien (14) verlaufenden Querschnitt zwei beidseitig an den Durchtrittskanal (13) angrenzende Bereiche (19) aufweist, wobei die Bereiche (19) jeweils abschnittsweise durch elektrisch leitende Wandabschnitte (20) begrenzt sind, und wobei die Innenkontur der genannten Wandabschnitte (20) von der Form eines Kreisbogens abweicht.

3. Mikrowellenhohlraumresonator (8) gemäß dem vorangegangenen Anspruch, **dadurch gekennzeichnet, dass** die Innenkontur der elektrisch leitenden Wandabschnitte (20) in dem genannten Querschnitt zumindest abschnittsweise geradlinig verläuft.

4. Mikrowellenhohlraumresonator (8) gemäß Anspruch 2 oder 3, **dadurch gekennzeichnet, dass** die Innenkontur der elektrisch leitenden Wandabschnitte (20) der beiden Bereiche (19) in dem genannten Querschnitt jeweils eine C-förmige Form aufweist.

5. Mikrowellenhohlraumresonator (8) gemäß einem der Ansprüche 2 bis 4, **dadurch gekennzeichnet, dass** die Innenkontur der elektrisch leitenden Wandabschnitte (20) der beiden Bereiche (19) in dem genannten Querschnitt bezüglich des Durchtrittskanals (13) spiegelsymmetrisch ausgebildet ist.

6. Mikrowellenhohlraumresonator (8) gemäß einem der Ansprüche 2 bis 5, **dadurch gekennzeichnet, dass** die elektrisch leitenden Wandabschnitte (20) der beiden Bereiche (19) der Resonatorkammer (7) in dem genannten Querschnitt jeweils eine Unterbrechung aufweisen, wobei die Unterbrechungen jeweils von einem elektrisch nicht leitenden und den Durchtrittskanal (13) zumindest abschnittsweise begrenzenden Kanalwandungsabschnitt (21) überbrückt sind.

7. Mikrowellenhohlraumresonator (8) gemäß dem vorangegangenen Anspruch, **dadurch gekennzeichnet, dass** die elektrisch nicht leitenden Kanalwandungsabschnitte (21) in dem genannten Querschnitt eine parallel zur Transportrichtung (T) verlaufende Breite (B) aufweisen, die kleiner ist als die senkrecht zur Transportrichtung (T) und parallel zu den genannten Linien (14) verlaufende maximale Höhe (H) der Resonatorkammer (7).

8. Mikrowellenhohlraumresonator (8) gemäß dem vorangegangenen Anspruch, **dadurch gekennzeichnet, dass** die maximale Höhe (H) der Resonatorkammer (7) einen Betrag aufweist, der zwischen 80 mm und 200 mm, bevorzugt zwischen 100 mm und 180 mm, liegt.

9. Mikrowellenhohlraumresonator (8) gemäß Anspruch 7 oder 8, **dadurch gekennzeichnet, dass** die in Anspruch 7 genannte Breite (B) der elektrisch nicht leitenden Kanalwandungsabschnitte (21) einen Betrag aufweist, der zwischen 20 mm und 80 mm, bevorzugt zwischen 30 mm und 70 mm, liegt.

10. Mikrowellenhohlraumresonator (8) gemäß einem der Ansprüche 2 bis 9, **dadurch gekennzeichnet, dass** die Innenkonturen der beiden Bereiche (19) der Resonatorkammer (7) in dem genannten Querschnitt jeweils die Form eines Polygons aufweisen, wobei ein Teil des Polygons durch die Innenkontur einzelner elektrisch leitender Wandabschnitte (20) und der restliche Teil des Polygons durch einen elektrisch nicht leitenden Kanalwandungsabschnitt (21) gebildet ist.

11. Mikrowellenhohlraumresonator (8) gemäß einem der Ansprüche 2 bis 10, **dadurch gekennzeichnet, dass** die Innenkonturen der beiden Bereiche (19) der Resonatorkammer (7) jeweils quader- oder trogförmig ausgebildet sind.

12. Spinnereivorbereitungsmaschine (1), insbesondere in Form einer Karde, Kämmmaschine oder Strecke, **dadurch gekennzeichnet, dass** sie zumindest einen Mikrowellenhohlraumresonator (8) gemäß einem der vorangegangenen Ansprüche aufweist.
